# EUROPEAN PATENT APPLICATION

(11) **EP 4 212 616 A1**
(43) Date of publication of application: **19.07.2023**
(21) Application number: 20953401.5
(22) Date of filing: 14.09.2020
(51) Int. Cl.: C12N 5/0775, C07K 14/47, A61K 35/28, A61K 48/00, A61P 25/28

(54) **METHOD FOR PREPARING STEM CELLS HAVING IMPROVED ENGRAFTMENT CAPABILITY**

(71) Applicant: Generoath Co., Ltd., Seoul 04168 (KR)
(72) Inventor: CHOI, Jin Woo, Seoul 07028 (KR)
(74) Representative: König Szynka Tilmann von Renesse Patentanwälte Partnerschaft mbB Düsseldorf
(86) International application number: PCT/KR2020/012375
(87) International publication number: WO 2022/054999

(57) **Abstract**

The present invention relates to a method for producing stem cells with increased engraftability and various uses thereof. Stem cells introduced with the exon 2-deleted AIMP2 variant of the present invention showed excellent engraftability as engraftment protein expression, cell number, cell viability, and apoptosis resistance increased. Therefore, the stem cells of the present invention can be activated in a form suitable for transplantation and administration with excellent human-body compatibility and economically manufactured in large quantities. Therefore, they are appropriate as cell therapy products and can be used in related industries.

## Description

### Field of the Invention

The present invention relates to a method for producing stem cells with increased engraftability and various uses thereof.

### Background Art

Stem cells, collectively referring to undifferentiated cells in a pre-differentiated stage, can differentiate into various cells constituting biological tissues and obtainable from each embryo, fetus, and adult tissue. Stem cells differentiate into specific cells by differentiation stimuli (environments). Unlike other cells where cell division is arrested after differentiation, stem cells proliferate by producing the same cells (self-renewing) as themselves through cell division. They are characterized by unique plasticity in differentiation because they can be differentiated into various cells depending on different environments or differentiation stimuli.

Stem cells are primarily classified into pluripotent embryonic stem cells (ES cells) obtained from embryos with the totipotent ability to differentiate into all cell types and multipotent adult stem cells obtained from each tissue type. The inner cell mass of the blastocyst, which is the early stage of embryonic development, is the part that will form the fetus in the future. Embryonic stem cells formed from this inner cell mass are totipotent, theoretically being able to differentiate into cells of all tissues constituting an organism. That is, embryonic stem cells are undifferentiated cells capable of unlimited proliferation and can differentiate into all cells. On the other hand, adult stem cells are cells that can differentiate into multiple cells within their lineages.

As described above, stem cells are either multi- or pluripotent, presenting significant potential in cell therapy in that they can induce differentiation into necessary cells in damaged tissues. Still, it is challenging to find widely and stably successful examples in clinical applications due to the unsatisfactory survival rate after transplantation at the current development level. Thus, it is necessary to activate the stem cells in a state suitable for transplantation by giving appropriate stimulation to the stem cells or changing their characteristics.

On the other hand, AIMP2 is an alternative splicing variant of AIMP2, a tumor suppressor involved in apoptosis in many ways, known to inhibit apoptosis by inhibiting the function of AIMP2. AIMP2/p38 facilitates the ubiquitination of TRAF2 and regulates TNF-α-induced apoptosis. AIMP2, a splicing variant of AIMP2/p38, acts as a competitive inhibitor with AIMP2, inhibits TRAF2 ubiquitination, and inhibits TNF-α-induced apoptosis, thereby inhibiting the expression of Cox-2, a marker of tumorigenesis and inflammation.

In addition, AIMP2 has been previously reported as a lung cancer inducer. The previous study confirmed that AIMP2, a variant of AIMP2, occurs in cancer cells in large numbers and causes cancer by interfering with the anti-cancer function of AIMP2. It was found that when AIMP2 was expressed in normal cells, cell cancerization proceeded. On the contrary, when the generation of AIMP2 was suppressed, cancer growth was suppressed, resulting in a therapeutic effect.

Accordingly, the present inventors introduced a variant of AIMP2 into stem cells. As a result, the present invention confirmed the effect of higher engraftability, such as increased engraftment protein expression, cell count, cell viability, and apoptosis resistance.

### Specific Description of the Invention

### Technical Challenges

An object of the present invention may provide a method to produce stem cells with increased engraftability, which includes introducing an exon 2-deleted AIMP2 variant into stem cells.

In addition, an object of the present invention may provide stem cells with increased engraftability into which an exon 2-deleted AIMP2 variant is introduced.

In addition, an object of the present invention may provide a composition for enhancing the engraftability of stem cells containing an exon 2-deleted AIMP2 variant.

In addition, an object of the present invention may provide a method for increasing engraftability of stem cells, which includes introducing an exon 2-deleted AIMP2 variant into stem cells.

In addition, an object of the present invention may provide a composition for preventing or treating degenerative brain diseases comprising stem cells as an active ingredient, into which an exon 2-deleted AIMP2 variant is introduced.

In addition, an object of the present invention may provide a stem cell transplantation aid comprising a vector containing an exon 2-deleted AIMP2 variant or a polynucleotide encoding the same.

### Solutions for the Challenges

In order to achieve the above objects, the present invention provides a method for producing stem cells with increased engraftability, which includes introducing an exon 2-deleted AIMP2 variant into stem cells.

In addition, the present invention provides stem cells with increased engraftability into which an exon 2-deleted AIMP2 variant is introduced.

In addition, the present invention provides a composition for enhancing the engraftability of stem cells containing an exon 2-deleted AIMP2 variant.

In addition, the present invention provides a method for increasing the engraftability of stem cells, which includes introducing an exon 2-deleted AIMP2 variant into stem cells.

In addition, the present invention provides a composition for preventing or treating degenerative brain diseases comprising stem cells as an active ingredient, into which an exon 2-deleted AIMP2 variant was introduced.

In addition, the present invention provides a stem cell transplantation aid comprising a vector containing an exon 2-deleted AIMP2 variant or a polynucleotide encoding the same.

### Effects of the Invention

In the present invention, stem cells into which the exon 2-deleted AIMP2 variant was introduced showed excellent engraftability as engraftment protein expression, cell number, cell viability, and apoptosis resistance increased. The stem cells of the present invention can be activated in a form suitable for transplantation or administration with excellent human-body compatibility and economically manufactured in large quantities. Therefore, they are appropriate as cell therapy products and can be used in related industries.

### Brief Description of the Figures

FIG. 1 confirms the accumulation of cell numbers over culture days of AAV-DX2-infected stem cells relative to the control AAV-GFP-infected stem cells.
FIG. 2 confirms the cell index of AAV-DX2-infected stem cells relative to the control AAV-GFP-infected stem cells over time (0, 4, and 7 hours).
FIG. 3 confirms the level of apoptosis of AAV-DX2-infected stem cells of the present invention relative to the control AAV-GFP-infected stem cells under different apoptosis-inducing conditions (i.e., TNF-alpha+ CHX or H2O2).
FIG. 4 confirms bone marrow-derived mesenchymal stem cells (bmMSC) isolated from mouse femur and tibia.
FIG. 5 determines the viral infection titer of AAV-DX2:
   Con: DX2 expression in untreated cells;
   GFP: DX2 expression in cells treated with AAV-GFP at 10000 MOI; and
   GAPDH: a gene for quantifying the expression of DX2.
FIG. 6 confirms DX2 expression at the mRNA level in mouse bone marrow-derived mesenchymal stem cells (bmMSC) in which DX2 is overexpressed.

### Best Mode for Carrying Out the Invention

Hereinafter, the present invention shall be described in detail as an embodiment of the present invention with reference to the attached figures. The following embodiments are presented as examples of the present invention. A detailed description may be omitted, provided that a detailed description of a well-known technology or configuration well known to those skilled in the art may unnecessarily obscure the gist of the present invention. The present invention is not limited thereby. The present invention is capable of various modifications and applications within the range of equivalents interpreted from the claims described later and therefrom.

Unless otherwise indicated, nucleic acids are written in the 5'→3' direction from left to right.

Numerical ranges recited within the Specification are inclusive of the numbers defining the range and include each integer or any non-integer fraction within the defined range.

In addition, the terminology used in this specification intends to appropriately express a preferred embodiment of the present invention, which may vary according to the user's or operator's intention or customs of the field to which the present invention belongs. In addition, the terminology used in this Specification intends to appropriately express a preferred embodiment of the present invention, which may vary according to the user's or operator's intention or customs of the field to which the present invention belongs. Hence, definitions of these terms will have to be made based on the context throughout this Specification. In the entire Specification, the expression that a certain part "includes" a certain component means that it may further include other components without excluding others unless otherwise stated.

This Specification uses conventional one-letter and three-letter codes for naturally occurring amino acids and generally accepted three-letter codes for other amino acids (e.g., Aib (α-aminoisobutyric acid), Sar (N-methylglycine)). Amino acids, referred to by abbreviations in the present invention, are described according to the IUPAC-IUB nomenclature as follows:
Alanine: A, Arginine: R, Asparagine: N, Aspartic acid: D, Cysteine: C, Glutamic acid: E, Glutamine: Q, Glycine: G, Histidine: H, Isoleucine: I, Leucine: L, Lysine: K, Methionine: M, Phenylalanine: F, Proline: P, Serine: S, Threonine: T, Tryptophan: W, Tyrosine: Y, and Valine: V.

The present invention provides a method for producing stem cells with increased engraftability, which include introducing an exon 2-deleted AIMP2 variant into stem cells.

In the present invention, the term "engraftability" refers to the ability of transplanted stem cells to differentiate subsequent cells; cells produced in the body after transplantation; or implanted cells to replace lost or damaged cells. Enhanced engraftability may have the advantages of, for example, a reduction in the number of effective transplanted stem cells, a shortening of the stem cell culture period in vitro, an increase in the safety of stem cells, and a reduction in manufacturing cost.

The stem cells include one selected from the group consisting of embryonic stem cells, adult stem cells, induced pluripotent stem cells, embryonic germ cells, and embryonic tumor cells, but not limited thereto. The adult stem cell is one selected from the group consisting of hematopoietic stem cells, neural stem cells, and mesenchymal stem cells but is not limited thereto.

The hematopoietic stem cells may be isolated from umbilical cords or bone marrow, the neural stem cells may be isolated from brain tissue, and mesenchymal stem cells may be extracted from bone marrow, adipose tissue, or umbilical cords but are not limited thereto.

The introduction may use a recombinant vector containing an exon 2-deleted AIMP2 variant.

The term "culture" in the present invention means to grow microorganisms or cells under appropriate artificially controlled environmental conditions.

The stem cells can grow in a standard medium. In order to grow specific microorganisms or cells, the medium may contain nutrients required for the culture objects, i.e., microorganisms or cells to be cultured, and substances for particular purposes may be added. The medium is also referred to as an incubator or a culture medium and includes all of a natural medium, a synthetic medium, or a selective medium.

The medium used for culture must appropriately meet the requirements of a specific strain while controlling temperature, pH, etc., in a standard medium containing an appropriate carbon source, nitrogen source, amino acid, vitamin, etc. A mixed sugar of glucose and xylose is used as the primary carbon source. Other applicable carbon sources include sugars and carbohydrates (e.g., sucrose, lactose, fructose, maltose, starch, cellulose, etc.), oils and fats (e.g., soybean oil, sunflower oil, castor oil, coconut oil, etc.), fatty acids (e.g., palmitic acid, stearic acid, linoleic acid, etc.), alcohols (e.g., glycerol, ethanol, etc.), and organic acids (e.g., acetic acid, etc.). These substances may be used individually or as a mixture. Applicable nitrogen sources include inorganic nitrogen sources (e.g., ammonia, ammonium sulfate, ammonium chloride, ammonium acetate, ammonium phosphate, ammonium carbonate, and ammonium nitrate) and organic nitrogen sources (e.g., amino acids and peptones, including AAA, NZ-amines; meat extract; yeast extract; malt extract; corn steep liquor; casein hydrolysate; fish or degradation products thereof; defatted soybean cake or degradation products thereof). These nitrogen sources may be used individually or in combination. The medium may contain monopotassium phosphate, dipotassium phosphate, and corresponding sodium-containing salts as a phosphorus source. Applicable phosphorus sources include potassium dihydrogen phosphate, dipotassium hydrogen phosphate, or corresponding sodium-containing salts. In addition, as inorganic compounds, sodium chloride, calcium chloride, iron chloride, magnesium sulfate, iron sulfate, manganese sulfate, and calcium carbonate may be used. Finally, essential growth substances such as amino acids and vitamins may be used in addition to the above substances.

In addition, precursors suitable for the culture medium may be used. The above substances may be added in a batch, fed-batch, or continuous manner by a method appropriate to the culture product in the culture process but are not particularly limited thereto. Essential compounds such as sodium hydroxide, potassium hydroxide, ammonia, or acid compounds such as phosphoric acid or sulfuric acid can be used appropriately to adjust the culture product's pH.

For purposes of the present invention, the sequence of the AIMP2 protein as an exon 2-deleted AIMP2 variant (312aa version:AAC50391.1 or GI:1215669; 320aa version: AAH13630.1, GI:15489023, BC013630.1) is described in the literature (312aa version: Nicolaides,N.C., Kinzler,K.W. and Vogelstein,B. Analysis of the 5' region of PMS2 reveals heterogeneous transcripts and a novel overlapping gene, Genomics 29 (2), 329-334 (1995)/ 320 aa version: Generation and initial analysis of more than 15,000 full-length human and mouse Cdna sequences, Proc. Natl. Acad. Sci. U.S.A. 99 (26), 16899-16903 (2002)).

In the present invention, the term "AIMP2 variant" results from partial or total loss of exon 2 among exons 1 to 4. The variant forms a heterodimer with the AIMP2 protein to interfere with the normal function of AIMP2.

The AIMP2 variant may be a nucleotide sequence represented by SEQ ID NO: 1 and an amino acid sequence represented by SEQ ID NO: 2.

Variants of the above sequences are included within the scope of the present invention. It includes a variant capable of functionally the same action as the nucleotide sequence of SEQ ID NO: 1 while functional equivalents of the nucleotide sequence constituting SEQ ID NO: 1 of the present invention, for example, some of the nucleotide sequences of SEQ ID NO: 1 of the present invention is modified by deletion, substitution or insertion. Specifically, it may include a base sequence having a sequence homology of at least 70%, more preferably at least 80%, even more preferably at least 90%, and most preferably at least 95% with the base sequence of SEQ ID NO: 1. The "percentage of sequence homology" for a base sequence is determined by comparing the two optimally aligned sequences with the region of comparison. Part of the base sequence in the comparison region may contain additions or deletions (i.e., gaps) compared to the reference sequence (not including additions or deletions) for an optimal alignment of the two sequences.

In addition, the scope of the protein in the present invention includes a protein having the amino acid sequence represented by SEQ ID NO: 2 and functional equivalents of the protein. "Functional Equivalent" means at least 70% or more, preferably 80% or more, more preferably 90% or more, even more preferably 95% or more sequence homology to the amino acid sequence represented by SEQ ID NO: 2 as a result of amino acid additions, substitutions or deletions. It refers to a protein exhibiting the same physiological activity as the amino acid protein represented by SEQ ID NO: 2.

Proteins of the present invention include proteins having the native amino acid sequence as well as amino acid sequence variants thereof within the scope of the present invention.

A variant of proteins of the present invention refers to a protein having a sequence different from the natural amino acid sequence by deletion, insertion, the non-conservative or conservative substitution of one or more amino acid residues, or a combination thereof. Amino acid substitution in proteins and peptides that do not entirely alter the molecule activity are known in the art (H. Neurath, R.L. Hill, The Proteins, Academic Press, New York, 1979).

The protein or its variants can be extracted from nature, synthesized (Merrifleld, J. Amer. chem. Soc. 85:2149-2156, 1963), or produced by genetic recombination methods based on DNA sequences (Sambrook et al., Molecular Cloning, Cold Spring Harbor Laboratory Press, New York, USA, 2nd Edition, 1989).

The amino acid variation is based on the relative similarity of amino acid side chain substituents, such as hydrophobicity, hydrophilicity, charge, size, etc. By analysis of the size, shape, and type of amino acid side chain substituents, arginine, lysine, and histidine are all positively charged residues; alanine, glycine, and serine have similar sizes; phenylalanine, tryptophan, and tyrosine have similar shapes.

Therefore, based on these considerations, arginine, lysine, and histidine; alanine, glycine, and serine; and phenylalanine, tryptophan, and tyrosine are biologically functional equivalents.

In introducing variations, the hydropathic index of amino acids can be considered. Each amino acid is assigned a hydrophobicity index according to its hydrophobicity and charge: isoleucine (+4.5); valine (+4.2); leucine (+3.8); phenylalanine (+2.8); cysteine/cysteine (+2.5); methionine (+1.9); alanine (+1.8); glycine (-0.4); threonine (-0.7); serine (-0.8); tryptophan (-0.9); Tyrosine (-1.3); proline (-1.6); histidine (-3.2); glutamate (-3.5); glutamine (-3.5); aspartate (-3.5); asparagine (-3.5); lysine (-3.9); and arginine (-4.5).

The hydrophobic amino acid index is critical in conferring the interactive biological function of proteins. It is known that similar biological activities can be obtained only when amino acids with similar hydropathic indexes are substituted. When introducing variations with reference to the hydropathic index, substitutions are made between amino acids that exhibit a hydropathic index difference, preferably within ± 2, more preferably within ± 1, and even more preferably within ± 0.5.

On the other hand, it is also well known that substituting amino acids with similar hydrophilicity values results in proteins having equivalent biological activity. As disclosed in U.S. Patent No. 4,554,101, the following hydrophilicity values are assigned to each amino acid residue: arginine (+3.0); lysine (+3.0); asphaltate (+3.0±1); glutamate (+3.0±1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4).

When introducing variations with reference to the hydrophilicity value, substitutions are made between amino acids that exhibit hydrophilicity value difference, preferably within ± 2, more preferably within ± 1, and still more preferably within ± 0.5.

Amino acid substitutions in proteins that do not entirely alter the molecule activity are known in the art (H. Neurath, R.L. Hill, The Proteins, Academic Press, New York, 1979). The most commonly occurring substitutions are exchanges between amino acid residues: Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly.

The present invention provides a recombinant vector for increasing stem cell engraftability with an exon 2-deleted AIMP2 variant.

In the present invention, the term "recombinant vector" refers to a vector capable of expressing a target protein or target RNA in a suitable host cell and a genetic construct containing essential regulatory elements operably linked to express a gene insert.

In the present invention, the term "operably linked" refers to a functional linkage between a nucleic acid expression control sequence and a nucleic acid sequence encoding a protein or RNA of interest to perform a general function. For example, a promoter and a nucleic acid sequence encoding a protein or RNA may be operably linked to affect the expression of the encoding nucleic acid sequence. Operational linkages with recombinant vectors can be made using genetic recombination techniques well known in the art and uses enzymes commonly known in the art for site-specific DNA cleavage and ligation.

The vector system of the present invention can be constructed through various methods known in the art. A related specific method is disclosed in Sambrook et al. (2001), Molecular Cloning, A Laboratory Manual, Cold Spring Harbor Laboratory Press. This literature is incorporated herein by reference.

The vector of a present invention can typically be constructed as a vector for cloning or as a vector for expression. In addition, the vector of the present invention may be built using prokaryotic or eukaryotic cells as a host. When the vector of the present invention is an expression vector and uses a prokaryotic cell as a host, it generally contains a strong promoter capable of promoting transcription (e.g., tac promoter, lac promoter, lacUV5 promoter, lpp promoter, pLλ promoter, pRλ promoter, rac5 promoter, amp promoter, recA promoter, SP6 promoter, trp promoter, and T7 promoter, etc.), a ribosome binding site for initiation of translation, and a transcription/translation termination sequence. When E. coli is used as the host cell (e.g., HB101, BL21, DH5α, etc.), the promoter and operator regions (Yanofsky, C. (1984), J. Bacteriol., 158:1018-1024) of the E. coli tryptophan biosynthetic pathway and the leftward promoter (pLλ promoter, Herskowitz, I. and Hagen, D. (1980), Ann. Rev. Genet., 14:399-445) of phage λ can be used as regulatory regions.

On the other hand, the vector that can be used in the present invention may be one or more selected from the group consisting of viral vectors, linear DNA, and plasmid DNA.

In the present invention, the term "viral vector" refers to a viral vector capable of delivering a therapeutic gene or genetic material to a desired cell, tissue, and/or organ.

The viral vector may include one or more selected from the group consisting of adenovirus, adeno-associated virus, lentivirus, retrovirus, HIV (human immunodeficiency virus), MLV (murine leukemia virus), ASLV (avian sarcoma/leukosis), SNV (spleen necrosis virus), RSV (Rous sarcoma virus), MMTV (mouse mammary tumor virus) and herpes simplex virus, but is not limited thereto.

Retroviruses can integrate into the host cell's genome and are harmless to the human body while inhibiting the function of normal cells when integrated. It is characterized by infecting various cells, proliferating easily, being capable of accommodating foreign genes of about 1 to 7 kilobases, and generating replication-deficient viruses.

Nevertheless, retroviruses have disadvantages in that they are difficult to infect cells after mitosis, are difficult to transfer genes in vivo, and must always propagate somatic tissue in vitro. In addition, retroviruses can integrate into proto-oncogenes, which carry the risk of mutation and cause cell death.

On the other hand, adenovirus has several advantages as a cloning vector. This medium-sized virus is replicable in the cell nucleus; clinically non-toxic; stable even when a foreign gene is inserted; invokes no gene rearrangements or losses; can transform eukaryotes; expressed stably and at a high level even when integrated into the host cell chromosome. A suitable host cell for adenovirus is a cell that causes myeloma, such as human hematopoietic and lymphatic cells. However, its linear DNA structure makes it difficult to multiply and challenging to recover infected viruses with a low infection rate. In addition, expression of the transferred gene is highest after 1 to 2 weeks, and expression is maintained only for 3 to 4 weeks in some cells. Also another problem is its high immunogenicity.

Adeno-associated virus (AAV), also called adeno-satellitovirus, is recently preferred because it can supplement the above concerns and has many advantages as a gene therapy agent. Adeno-associated virus particles are about 20 nm in diameter and known to have little harm to the human body; therefore have been approved for marketing as gene therapy products in Europe.

The AAV is a single-stranded provirus that requires an auxiliary virus to replicate. The 4,680-bp AAV genome can be inserted into an infected cell's specific site on chromosome 19. The trans-gene is inserted into plasmid DNA linked by two inverted-terminal repeats (ITR) sequences and signal sequences of 145 bp each. AAV rep and cap parts are transfected with other plasmid DNA expressing them, and adenovirus is added as a helper virus. AAV has the advantages of a wide range of host cells that deliver genes, low immune side effects upon repeated administration, and a long gene expression period. Moreover, the AAV genome is safe to integrate into the host cell's chromosome and does not alter or rearrange the host's gene expression.

The adeno-associated virus is known to have four serotypes. Among many adeno-associated virus serotypes that can deliver a target gene, the most widely studied vector is adeno-associated virus serotype 2, currently used for clinical gene transfer in cystic fibrosis, hemophilia, and Canavan disease. Also, the potential of rAAV (recombinant adeno-associated virus) in cancer gene therapy has recently increased.

Adeno-associated virus serotype 2 is also used in the present invention. An appropriate viral vector can be selected and applied but is not limited thereto.

In addition, When the vector of the present invention is an expression vector and uses a prokaryotic cell as a host, a promoter derived from the genome of a mammalian cell (e.g., metallothionein promoter) or a promoter derived from a mammalian virus (e.g., adenovirus late promoter, vaccinia virus 7.5K promoter, SV40 promoter, cytomegalovirus promoter, and HSV promoter) may be used. Specifically, it may include at least one selected from promoters selected from the group consisting of retroviral LTR, cytomegalovirus (CMV) promoter, Rous sarcoma virus (RSV) promoter, MMT promoter, EF-1 alpha promoter, UB6 promoter, chicken beta-actin promoter, CAG promoter, RPE65 promoter, and opsin promoter, but is not limited thereto, generally with a polyadenylation sequence as a transcription termination sequence.

Vectors of the present invention may be fused with other sequences as needed to facilitate protein purification, including, for example, glutathione S-transferase (Pharmacia, USA), maltose binding protein (NEB, USA), FLAG (IBI, USA), and 6x His (hexahistidine; Quiagen, USA) or the like but not limited thereto. In addition, the expression vector of the present invention may contain an antibiotic resistance gene commonly used in the art as a selection marker. Examples include genes for resistance to ampicillin, gentamicin, carbenicillin, chloramphenicol, streptomycin, kanamycin, geneticin, neomycin, and tetracycline.

In addition, the recombinant vector of the present invention can be introduced in various ways: CaCl2 method (Cohen, S.N. et al. (1973), Proc. Natl. Acac. Sci. USA, 9:2110-2114); Hanhan method (Cohen, S.N. et al. (1973), Proc. Natl. Acac. Sci. USA, 9:2110-2114; and Hanahan, D. (1983), J. Mol. Biol., 166:557-580); and the electroporation method (Dower, W.J. et al. (1988), Nucleic Acids Res., 16:6127-6145) and the like.

In addition, the present invention provides stem cells with increased engraftability into which an exon 2-deleted AIMP2 variant is introduced.

The stem cells of the present invention may additionally include a pharmaceutical composition.

Specifically, the stem cells of the present invention can be used as a treatment for degenerative brain diseases. The degenerative brain disease may be one or more selected from the group consisting of Alzheimer's disease, Parkinson's disease, amyotrophic lateral sclerosis, retinal degeneration, mild cognitive impairment, multiple-infarction dementia, frontotemporal dementia, dementia with Lewy bodies, Huntington's disease, neurodegenerative disease, metabolic brain disease, depression, epilepsy, multiple sclerosis, corticobasal degeneration, multiple system atrophy, progressive supranuclear palsy, dentatorubropallidoluysian atrophy, spinocerebellar ataxia, primary lateral sclerosis, spinal muscular atrophy, and stroke.

The pharmaceutical composition of the present invention may further include an adjuvant in addition to the active ingredient. As long as the adjuvant is known in the art, any adjuvant may be used without limitation. For example, Freund's complete adjuvant or incomplete adjuvant may be further included to increase immunity.

A pharmaceutical composition according to the present invention may be prepared in a form incorporating an active ingredient into a pharmaceutically acceptable carrier. Here, the pharmaceutically acceptable carrier includes carriers, excipients, and diluents commonly used in the pharmaceutical field. Pharmaceutically acceptable carriers that can be used in the pharmaceutical composition of the present invention include, but are not limited to, lactose, dextrose, sucrose, sorbitol, mannitol, xylitol, erythritol, maltitol, starch, gum acacia, alginate, gelatin, calcium phosphate, calcium silicate, cellulose, methyl cellulose, polyvinyl pyrrolidone, water, methyl hydroxybenzoate, propyl hydroxybenzoate, talc, magnesium stearate, and mineral oil.

The pharmaceutical compositions of the present invention may be formulated and used in the form of oral formulations such as powders, granules, tablets, capsules, suspensions, emulsions, syrups, aerosols, external preparations, suppositories, or sterile injection solutions depending on standard methods, respectively.

Diluents or excipients such as commonly used fillers, extenders, binders, wetting agents, disintegrants, and surfactants may be used in the formulation. Solid dosage forms for oral administration include tablets, pills, powders, granules, and capsules. Such a solid preparation may be prepared by mixing active ingredients with at least one or more excipients, for example, starch, calcium carbonate, sucrose, lactose, gelatin, and the like. In addition to simple excipients, lubricants such as magnesium stearate and talc may also be used. Liquid formulations for oral administration include suspensions, internal solutions, emulsions, syrups, and the like. In addition to commonly used diluents such as water and liquid paraffin, various excipients such as wetting agents, sweeteners, aromatics, and preservatives may be included. Formulations for parenteral administration include sterilized aqueous solutions, non-aqueous solvents, suspensions, emulsions, freeze-dried formulations, and suppositories. Propylene glycol, polyethylene glycol, vegetable oils such as olive oil, and injectable esters such as ethyl oleate may be used as non-aqueous solvents and suspending agents. Witepsol, Tween 61, cocoa butter, laurinum, glycerogelatin, and the like may be used as a base for suppositories.

According to the present invention, a pharmaceutical composition may be administered to a subject by various routes. All modes of administration are contemplated, e.g., oral, intravenous, intramuscular, subcutaneous, and intraperitoneal injection.

The preferred dosage of the composition for treatment according to the present invention can be appropriately selected by those skilled in the art, although it varies depending on factors such as formulation method, administration method, patient's age, weight, gender, the severity of disease symptoms, food, administration time, administration route, excretion rate, and reaction sensitivity.

However, for the therapeutic effect, an ordinarily skilled physician can readily determine and prescribe an effective dosage for the desired treatment. For example, the therapeutic agent includes intravascular injection, subcutaneous fat injection, intramuscular injection, and direct injection into the brain ventricle or spinal cord using a microinjector. At this time, multiple injections and repeated administration are possible. The effective dose is 0.05 to 15 mg/kg vector per 1 kg of body weight for intravascular injection; 1 X 107 to 1 X 1011 virus particles (1 X 105 to 1 X 109 IU)/kg for recombinant viruses; 1 X 10 3 to 1 X 10 6 cells/kg for cells; preferably 0.1 to 10 mg/kg for vectors; 1 X 108 to 1 X 1010 particles (1 X 106 to 1 X108 IU)/kg for recombinant viruses; 1 X102 to 1 X 105 cells/kg for cells to be administered 2 to 3 times a week. The composition, as described above, is not necessarily limited thereto and may vary depending on the condition of the patient and the degree of onset of neurological disease. The effective dose for other subcutaneous fat, intramuscular injection, and direct administration to the affected area is 1 X 107 to 1 X 109 recombinant virus particles administered at intervals of 10 cm and can be administered 2 to 3 times a week. The composition, as described above, is not necessarily limited thereto and may vary depending on the condition of the patient and the degree of onset of neurological disease. More specifically, the pharmaceutical composition of the present invention contains 1 X 105 to 1 X 1015 PFU/mL of recombinant adeno-associated virus, and it is usually recommended to inject 1 X 1010 PFU once every other day for two weeks. Administration may be done once a day or in several divided doses.

The pharmaceutical composition may be formulated into various oral or parenteral dosage forms.

Formulations for oral administration include, for example, tablets, pills, hard and soft capsules, solutions, suspensions, emulsifiers, syrups, granules, and the like. These formulations may further include a diluent (e.g., lactose, dextrose, sucrose, mannitol, sorbitol, cellulose, and/or glycine) and a lubricant (e.g., silica, talc, stearic acid and its magnesium or calcium salts, and/or polyethylene glycol) in addition to the active ingredient. The tablets may contain a binder such as magnesium aluminum silicate, starch paste, gelatin, tragacanth, methylcellulose, sodium carboxymethylcellulose, and/or polyvinylpyrrolidone. It may also optionally contain disintegrants or effervescent mixtures and/or absorbents, colorants, flavors, and sweeteners, such as starch, agar, alginic acid, or sodium salt. The formulation may be prepared by standard mixing, granulating, or coating methods.

In addition, a typical formulation for parenteral administration is an injection formulation. Examples of the solvent for the preparation for injection include water, Ringer's solution, isotonic physiological saline, or suspension. Sterile, fixed oils of the above injectable preparations may be used as a solvent or suspension medium. Any bland fixed oil, including mono- or di-glycerides, may be employed.

In addition, the injection formulation may use a fatty acid such as oleic acid.

In addition, the present invention provides a composition for enhancing the engraftability of stem cells comprising a vector containing an exon 2-deleted AIMP2 variant or a polynucleotide encoding the same.

The vector may be a viral vector.

In addition, the present invention provides a method for increasing the engraftability of stem cells, which include introducing an exon 2-deleted AIMP2 variant into stem cells.

In addition, the present invention provides a composition for enhancing the engraftability of stem cells containing an exon 2-deleted AIMP2 variant.

A composition for enhancing the engraftability of stem cells may be a medium composition.

Common media for culturing stem cells are known in the art. These media contain salts, vitamins, buffers, energy sources, amino acids, and other substances. When stem cells are cultured, about 5 to 20% of animal-derived serum can be included to provide universal nutrients for the growth of cells to be cultured.

In addition, the present invention provides a composition for preventing or treating degenerative brain diseases comprising stem cells transduced with an exon 2-deleted AIMP2 variant as an active ingredient.

Specifically, the degenerative brain diseases may be one or more selected from the group consisting of Alzheimer's disease, Parkinson's disease, amyotrophic lateral sclerosis, retinal degeneration, mild cognitive impairment, Multi-infarct dementia, frontotemporal dementia, dementia with Lewy bodies, Huntington's disease, neurodegenerative disease, metabolic brain disease, depression, epilepsy, Multiple sclerosis, corticobasal degeneration, multiple system atrophy, progressive supranuclear palsy, dentatorubropallidoluysian atrophy, spinocerebellar ataxia, primary lateral sclerosis, spinal muscular atrophy, and stroke, but is not limited thereto.

In addition, the present invention provides a stem cell transplantation aid comprising a vector containing an exon 2-deleted AIMP2 variant or a polynucleotide encoding the same.

The transplantation aid may be administered simultaneously with or at the same time as the transplantation of stem cells and may promote the engraftment of transplanted stem cells.

In addition, the present invention provides a use of a vector containing an exon 2-deleted AIMP2 variant or a polynucleotide encoding the same to promote stem cell engraftability.

In addition, the present invention provides a method for promoting the transplantation of stem cells using a vector containing an exon 2-deleted AIMP2 variant or a polynucleotide encoding the same.

In the preferred embodiment of the present invention, the engraftability of stem cells was significantly improved both in vitro and in vivo when overexpressing the exon 2-deleted AIMP2 variant by infecting stem cells with the exon 2-deleted AIMP2 variant with a viral vector.

### Mode for Carrying Out the Invention

Hereinafter, the present invention shall be described in more detail as the preferred embodiment. However, the following preferred embodiment is only for embodying the contents of the present invention, and the present invention is not limited thereto.

### <Preferred Embodiment 1> Production of Stem Cells Infected with the AIMP2 Variant of the Present Invention

AIMP2 is one of the proteins involved in forming aminoacyl-tRNA synthetase (ARSs) and acts as a tumor suppressor. In order to construct a plasmid expressing the exon 2-deleted AIMP2 variant of AIMP2, the cDNA of the AIMP2 variant was cloned into pcDNA3.1-myc. After amplifying the AIMP2 variant from H322 cDNA using primers with EcoR1 and XhoI linkers, it was cloned into pcDNA3.1-myc using EcoR1 and Xho1 and inserted into the AAV vector plasmid. The AIMP2 variant inserted into AAV was named "AAV-DX2".

The AIMP2 variant of the present invention is represented by the nucleotide sequence of SEQ ID NO: 1 and the amino acid sequence of SEQ ID NO: 2.

The following steps were performed to introduce and culture the AAV-DX2 into adipose-derived stem cells.
(1) Up to 300 ml of lipoaspirate was put into a sterile medium bottle to perform lipoaspirate washing. Blood was removed with a 25 ml pipette, allowing the blood portion to settle. An equal volume of HBSS (w/ antibiotics, fungizone) was added, and the lid was tightly closed and shaken for 5 to 10 seconds. Then, after placing it on a flat surface for 1 to 5 minutes, HBSS was removed with a 50 ml pipette, and this process was repeated two more times. If the washing solution was red, washing was performed again.
(2) A 0.2% collagenase aqueous solution was prepared for collagenase digestion using HBSS. The washed adipose tissue was transferred to a T-160 flask, 40 ml of 0.2% collagenase aqueous solution was added, the lid was closed, and shaken for 5 to 10 seconds, then placed at 37 ° C for 1 to 2 hours and shaken every 15 minutes.
   While the ECM was being decomposed, Histopaque-1077 was put into two pieces of 15 ml each in a 50 ml tube, and 200 ml washing medium (2% FBS, antibiotics, fungizone in HBSS) was prepared. When the disassembly was completed, the adipose tissue had turned into a soup, and the reaction was terminated by adding FBS to 10% of the total volume.
(3) To separate the stromal vascular fraction (SVF) derived from adipose tissue, the enzymatically reacted adipose tissue was divided into 50 ml tubes and centrifuged at 400 g for 10 minutes at room temperature, leaving only the pellet (SVF).
(4) To separate stromal stem cells from the adipose tissue-derived cell matrix, red blood cell removal, unnecessary cells and tissue removal (cell mass and undigested tissue), stromal stem cell isolation, and stromal stem cell culture were performed.

Specifically, the erythrocyte removal was performed by adding 20 ml of RBC lysis buffer to the SVF pellet, floating it, and culturing it at room temperature for 10 minutes. After centrifugation at 300 g for 10 minutes, the lysis buffer was removed.

Then, to perform the unwanted cell and tissue removal (cell mass and undigested tissue), the SVF pellets were resuspended with 2 ml of wash medium. After each sample was collected in two 50 ml tubes, the cell clumps were allowed to settle by waiting for 1 minute. After passing each sample through 100 um and 40 um strainers, the volume was adjusted to the washing medium of each sample. The tube containing Histopaque was tilted at 45 degrees, and the sample was added slowly (at approximately 1 ml/sec). After centrifugation at 400 g for 30 minutes, the washing medium on the cells of the white line was removed. Then, carefully picking the white-lined cells, they were transferred to a new 50 ml tube and centrifuged at 300 g for 10 minutes with the same volume of wash medium (low brake setting). Then, 25 ml of washing medium was added, and only the pellet was left after washing.

Then, in order to separate stromal stem cells from endothelial cells and leukocytes by magnetic cell sorting, the pellets were suspended in 10 ml of column buffer (2 mM EDTA, 0.5% BSA in PBS) and collected in a 50 ml tube. After passing through a 40-um strainer to remove cell clumps, the number of cells was measured. After transferring to 15 ml and centrifuging at 300 g, 10 minutes, 4° C., column buffer was added to 10⁶/ml and suspended. Anti-CD31, 45 FITC-conjugated antibodies were added (10ul per 10⁷ cells) and incubated at 4°C for 15 minutes (careful for light, mixing once every 7 minutes). 2ml of column buffer per 10⁷ was added and centrifuged at 300g, 10 minutes, and 4°C. After removing the supernatant, 90 ul of column buffer was added per 10⁷, and 10 ul of MACS anti-FITC magnetic microbeads were added per 10⁷ cells. It was mixed well and cultured at 4°C for 15 minutes (mixed once every 7 minutes). Then, 2ml of column buffer was added per 10⁷, centrifugation was performed at 300g, 10 minutes, and 4°C, and the supernatant was removed. 500 ul of column buffer was added and floated, and the MACS LD column was mounted in a MidiMACS separation unit and washed with 2 ml of column buffer.

A 15 ml tube was installed to receive the solution passing through the column, the cell solution was put into the column, 1 ml of column buffer was put into the column twice, and the cells were collected. After that, the purity of the stem cells was confirmed under a fluorescence microscope. If the purity is lower than desired, the above process was repeated once and the number of cells was measured. After centrifugation at 300g, 10 minutes, 4 °C, it was frozen or cultured.

To culture stromal stem cells, 10⁵ stromal stem cells were suspended in 2 ml of OC2 medium (Without Phenol Red/FBS) and then inoculated into T-25 cells. After seven days, it was replaced with OC2 medium (Without Phenol Red/FBS) and cultured like normal cells (1:3).

### <Preferred Embodiment 2> Confirmation of Collagen and Fibronectin Expression of the AAV-DX2-Infected Stem Cells of the Present Invention

Initial adhesion and differentiation functions are essential when stem cells are administered to a subject. Collagen protein is vital as a component of the extracellular matrix (ECM), which plays a fundamental role in cell development. Fibronectin is a protein that binds to the extracellular matrix (ECM) and has functions of initial adhesion and cell differentiation of stem cells. Therefore, the expression of fibronectin and collagen 1 to 4 of "AAV-DX2", a stem cell infected with AIMP2 prepared in the preferred embodiment 1, was confirmed. The expression of fibronectin or collagen 1-4 was measured by purchasing a Human Fibronectin Quantikine ELISA Kit from R&D Systems and was performed according to the manufacturer's protocol.

As a result, the AAV-DX2-infected stem cells of the present invention significantly increased the expression of fibronectin and collagen 1-4, which suggests that the attachment and differentiation of the AAV-DX2-infected stem cells of the present invention are enhanced.

**[Table 1]**

| | **Expression (%)** |
|---|---|
| **Fibronectin** | 107% |
| **Collagen I** | 105% |
| **Collagen II** | 102% |
| **Collagen III** | 104% |
| **Collagen IV** | 110% |

### <Preferred Embodiment 3> Confirmation of Viability Increase by Culturing the AAV-DX2-infected Stem Cells of the Present Invention

When stem cells are administered to a subject, cell viability is essential to sustain the therapeutic effect. Therefore, to confirm the viability of the AAV-DX2-infected stem cells of the present invention, "AAV-GFP" was produced by introducing green fluorescent protein (GFP) into AAV instead of the AIMP2 variant as a control.

The cultured stem cells were infected with AAV-DX2 or AAV-GFP and cultured using MesenCult Proliferation Kit (Human) stem cell Technology. Cell viability was confirmed by measuring the number of cells according to culture days (0, 50, 100, 150). In addition, the cell index was confirmed over time (0, 4, 7 hours).

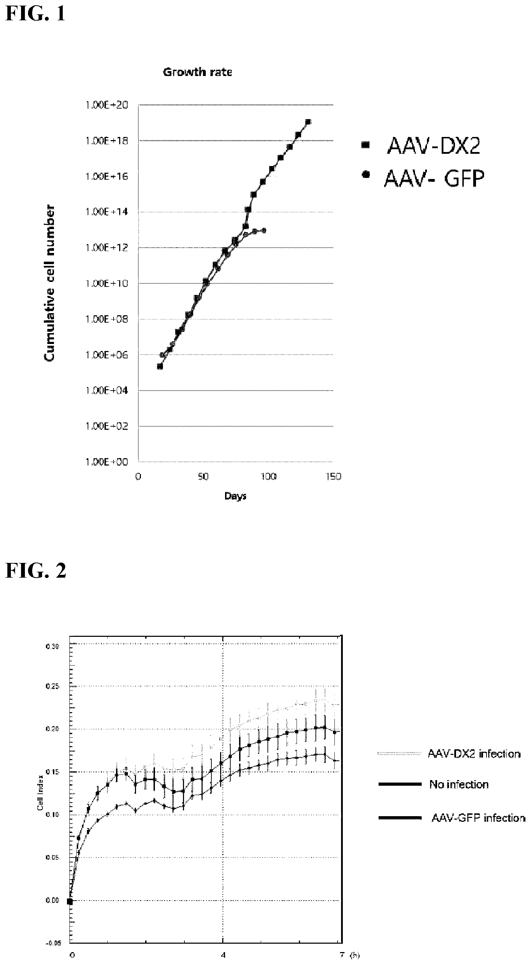

As a result, the AAV-DX2-infected stem cells of the present invention showed a more significant number of cell survival compared to the control AAV-GFP-infected stem cells (FIG. 1). Also, the number of control AAV-GFP-infected cells did not increase after a while over the culture days. However, AAV-DX2-infected stem cells of the present invention showed excellent cell viability by confirming that the number of cells continuously increased over the culture days (FIG. 1).

In addition, after verifying the cell index over time (0, 4, and 7 hours), the AAV-DX2-infected stem cells showed a higher cell index than the control group (FIG. 2).

Therefore, the results suggest that the AAV-DX2-infected stem cells of the present invention continuously increase cell number, thereby increasing stably and continuously maintaining viability when administered to a target subject.

### Confirmation of Apoptosis Resistance of the AAV-DX2-infected Stem Cells of the Present Invention

When stem cells are administered to a subject, various factors and conditions may induce apoptosis of these stem cells. Therefore, the apoptosis resistance of the AAV-DX2-infected stem cells of the present invention was confirmed under apoptosis-inducing conditions.

Specifically, control AAV-GFP-infected stem cells and AAV-DX2-infected stem cells of the present invention were treated each with TNF-alpha+CHX [TNF(30 ng/ml)+CHX(1 µg/ml), 24 h] or H2O2 (100 uM 24 h), which induces apoptosis. Then, PI (propidium iodide) staining was performed to see the cell cycle for each experimental group using FACS. Specifically, cells of each treatment group (70-80% culture per 100 mm Petri dish or homogenized tissue per 1X PBS) were prepared, and 1×10⁶ cells were centrifuged at 1500 rpm for 3 minutes. Next, they were washed with PBS and centrifuged under the same conditions. The cells were resuspended using 300ul PBS and treated with 700ul cold 100% EtOH for 15 minutes 4 times. After centrifugation under the same conditions, the cells were washed with PBS, resuspended, and stained with PI solution (50ug/ml PI, 0.1% sodium citrate, 0.3% NP-40, 50ug/ml RNase A), cultured at 4 degrees for 20-30 minutes, and then subjected to FACS without washing. The FACS conditions were E00/5.54/linear for FSC, 215/1.0/log for SSC, 150/1.0/log for FL1, 391/1.4/linear for FL2, 235/1.0/linear for FL3, 1.0/linear for FL2 area, and 1.5 /linear for FL2. Parameters of the FSC, SSC, and FL2 regions were replaced if necessary.

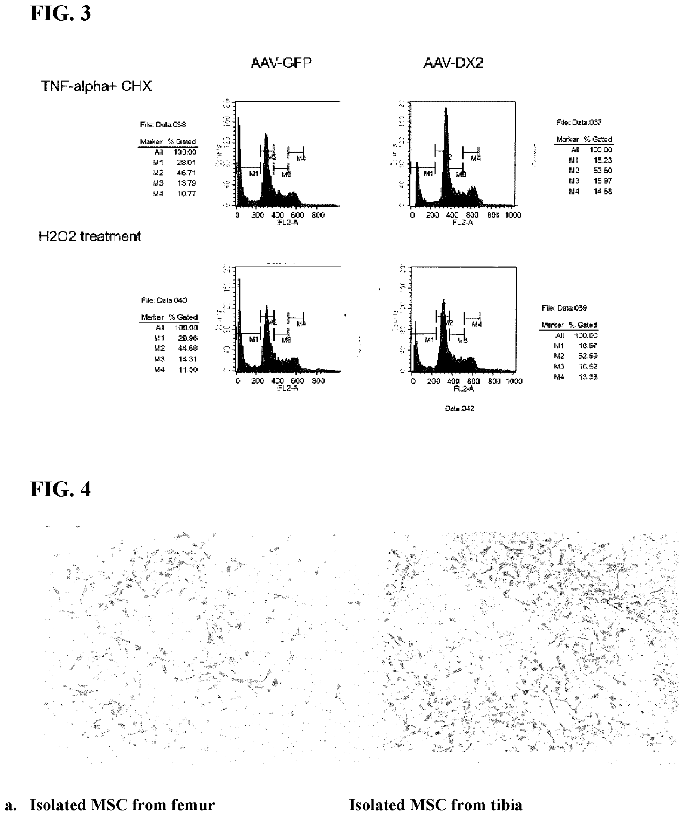

As a result, in each group treated with TNF-alpha+ CHX or H2O2, compared to the control AAV-GFP-infected stem cells, the AAV-DX2-infected stem cells of the present invention showed a difference of about two times or more in M1 where apoptosis appeared due to confirmed nuclear changes (FIG. 3). Therefore, stem cells infected with AAV-DX2 of the present invention were confirmed to have apoptosis resistance even under apoptosis-inducing conditions (FIG. 3).

### Confirmation of In Vivo Engraftability of the AAV-DX2-infected Stem Cells of the Present Invention

### 5-1. Isolation of bone marrow-derived mesenchymal stem cells (bmMSC)

Engraftability is important when stem cells are administered to a subject. In order to confirm the engraftability of stem cells by AAV-DX2 of the present invention in vivo, mouse mesenchymal stem cells were isolated. Specifically, both ends of the femur and tibia were cut after being separated from C57B1/6 8-week-old male mice. Then, cells containing bmMSC were taken using a 1ml syringe and diluted in 1x PBS solution. The diluted cells were collected by centrifugation at 900g for 5 minutes and washed twice with 1x PBS, and then suspended in 5 ml of DMEM (Dulbecco's Modified Eagle's medium) containing 10% FBS (fetal bovine serum), placed in a tube containing 5 ml of Percoll, and centrifuged at 1100 g for 30 minutes. After washing the cell layer separated by centrifugation three times with 10 ml of DMEM containing 10% FBS, the separated cells were cultured in 10% FBS/DMEM medium (FIG. 4).

### 5-2. Determination of AAV-DX2 Viral Infection Titer

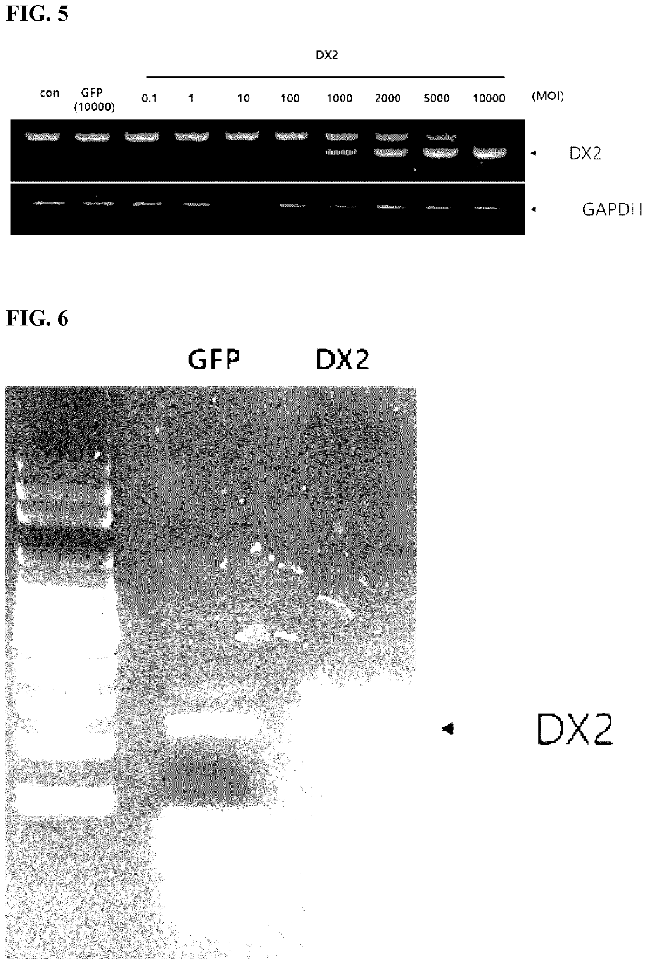

Before inducing overexpression of DX2 in mouse bmMSC isolated in the preferred embodiment 5-1, the viral infection titer of AAV-DX2 was determined. Specifically, after 24 hours of dispensing ARPE-1 cells at a concentration of 4x105 cells/6well, each cell was treated with AAV-DX2 virus at 0.1, 1, 10, 100, 1000, 2000, 5000, and 10000 MOI for 48 hours. Cells were prepared with TRIZOL, and the mRNA level of DX2 was measured by PCR using the primer sequences shown in Table 1 below. As a result, there was no change in DX2 expression in the control group, but the expression of DX2 increased as the concentration of AAV-DX2 increased. Above 2000 MOI, the expression of DX2 was saturated (FIG. 5), and therefore, the AAV-DX2 treatment concentration was determined at 1000 MOI.

**[Table 2]**

| | |
|---|---|
| Forward | 5'-CTGGCCACGTGCAGGATTACGGGG-3' |
| Reverse | 5'-AAGTGAATCCCAGCTGATAG-3' |

### 5-3. Induction of Overexpression of DX2 (AIMP2 variant)

Based on the above results, overexpression of DX2 was induced in mouse bmMSC isolated from preferred embodiment 5-1 using the AAV-DX2 virus. Specifically, mouse bmMSCs were seeded at 1×10⁶ cells/10 cm 2 and treated with 1000 MOI of AAV-DX2 (the control group was treated with 1000 MOI of AAV-GFP) for 48 hours to establish DX2-overexpressing mouse bmMSCs. Then, the expression of DX2 in mouse bmMSC was confirmed at the mRNA level by PCR (FIG. 6).

### Confirmation of In Vivo Engraftability of DX2 Overexpressing Cells

As in the preferred embodiment, in order to confirm the engraftability of mouse bmMSC overexpressing DX2 through AAV-DX2 infection in vivo, the GFP (control) or DX2-overexpressing mouse bmMSC was injected into mouse brain striatum, and survival and engraftment thereof were confirmed seven days later. Specifically, 8-week-old C57B1 / 6 male mice were anesthetized by intraperitoneal injection of 40 µl of ketamine mixture (a mixture of ketamine 50 mg/ml and Rompun (xylazine) 2.332 mg/ml in a 3:1 ratio.). Then the mice were fixed in stereotaxic with a tooth bar and an ear bar, and the mice's heads were leveled to be parallel. Then, the mouse head was disinfected, dissected, and a 1ml syringe was attached to the stereotaxic so that the injection needle hole was placed on the mouse brain bregma. After setting the AP ML DV value to 0, the syringe was set at the striatal coordinates AP: +0.5mm, ML: +1.8mm, and DV: -3.7mm. 2ul of 1×10⁶ cells (mouse bmMSC overexpressing GFP or DX2) were injected at a rate of 0.5 µl/min using a Hamilton syringe having a blunt end pierced through the skull of the mouse. After the injection, the syringe was rested for 3 3 minutes and slowly lifted at a rate of 1 mm per minute. Then, the injection was finished by suturing. At this time, DX2-overexpressing mouse bmMSC was injected into the right striatum, and GFP-expressing mouse bmMSC was injected into the left striatum. To confirm the viability and engraftability of the bmMSCs injected into both progenitors, tissue staining for CD44, a marker for mesenchymal stem cells, was performed. After the mice were sacrificed with isoflurane, they were perfused with 0.9% saline and 4% PFA (paraformaldehyde) to separate their brains. Isolated brains were fixed overnight in 4% PFA and dehydrated in 30% sucrose. After putting the dehydrated brain tissue in an OCT compound (optimum cutting temperature compound) and freezing it at -80 ° C, tissues were sectioned at 30 µm, stained with an anti-CD44 antibody, and the survival and engraftability of GFP-overexpressing mouse bmMSC and DX2-overexpressing mouse bmMSC were compared under a fluorescence microscope.

As a result, CD44 was not stained in GFP-overexpressing mouse bmMSC, whereas CD44 expression was observed in DX2-overexpressing mouse bmMSC, which showed that overexpression of the exon 2-deleted AIMP2 variant of AIMP2 improved the engraftability of stem cells in vivo.

## Claims

1. A method for producing stem cells with increased engraftability, comprising introducing an exon 2-deleted AIMP2 variant into stem cells.

2. The method of claim 1, wherein the stem cells are selected from embryonic stem cells, adult stem cells, induced pluripotent stem cells, embryonic germ cells, and embryonic tumor cells.

3. The method of claim 2, wherein the adult stem cells are selected from hematopoietic stem cells, neural stem cells, and mesenchymal stem cells.

4. The method of claim 1, wherein the introducing is performed using a recombinant vector containing a polynucleotide encoding the exon 2-deleted AIMP2 variant.

5. Stem cells with increased engraftability comprising an exon 2-deleted AIMP2 variant introduced into the stem cells.

6. The stem cells of claim 5, wherein stem cells have increased engraftability with a recombinant vector containing a polynucleotide encoding an exon 2-deleted AIMP2 variant.

7. The stem cells of claim 5, wherein the stem cells are selected from embryonic stem cells, adult stem cells, induced pluripotent stem cells, embryonic germ cells, and embryonic tumor cells.

8. A composition for enhancing engraftability of stem cells, comprising an exon 2-deleted AIMP2 variant.

9. A method of increasing engraftability of stem cells, comprising introducing an exon 2-deleted AIMP2 variant into stem cells.

10. A composition for preventing or treating a neurodegenerative disease, comprising stem cells transduced with an exon 2-deleted AIMP2 variant as an active ingredient.

11. A stem cell transplantation adjuvant comprising a vector containing an exon 2-deleted AIMP2 variant or a polynucleotide encoding the same.

12. Use of a vector containing an exon 2-deleted AIMP2 variant or a polynucleotide encoding the same to promote stem cell engraftability.

13. A method for promoting stem cell transplantation, comprising using a vector containing an exon 2-deleted AIMP2 variant or a polynucleotide encoding the same.
